# EUROPÄISCHE PATENTSCHRIFT

(11) **EP 1 359 212 B1**
(45) Veröffentlichungstag und Bekanntmachung des Hinweises auf die Patenterteilung: **19.12.2007**
(21) Anmeldenummer: 02009022.1
(22) Anmeldetag: 23.04.2002
(51) Int. Cl.: C11D 3/00, C11D 1/62, C11D 1/645, C11D 17/00, C11D 11/00

(54) **Tensidzubereitungen enthaltend mikroverkapselte Wirkstoffe**
Surfactant preparation comprising microencapsulated active ingredients
Preparations tensioactives comprenant des ingredients actifs micro-encapsules

(43) Veröffentlichungstag der Anmeldung: 05.11.2003
(73) Patentinhaber: Cognis IP Management GmbH, 40589 Düsseldorf (DE)
(72) Erfinder: Saborit, Luis, 08038 Barcelona (ES); Llosas Bigorra, Joaquim, Dr., 08203 Sabadell (ES)

(56) Entgegenhaltungen:
- EP-A- 0 539 025
- EP-A- 1 064 911
- EP-A- 1 064 912
- WO-A-01/62376
- DE-A- 10 008 305
- DE-A- 10 008 306
- DE-A- 10 041 004
- DE-A- 19 612 768

## Beschreibung

### Gebiet der Erfindung

Die Erfindung befindet sich auf dem Gebiet der mikroverkapselten Wirkstoffe und betrifft neue avivierende Zubereitungen mit speziellen Tensiden und Mikrokapseln, die Verwendung von kationischen Tensiden und chitosan-haltigen Mikrokapseln als Textil behandlungsmittel sowie ein Verfahren zur Ausrüstung von synthetischen oder natürlichen Fasern.

### Stand der Technik

Unter dem Begriff "Tragekomfort" werden gestiegene Anforderungen des Verbrauchers zusammengefasst, der sich nicht mehr allein damit zufrieden geben will, dass die von ihm unmittelbar auf der Haut getragene Wäsche weder kratzen noch Hautrötungen verursachen, sondern ganz umgekehrt erwartet, dass sie sich positiv auf den Zustand seiner Haut auswirken. Dabei kann es sich sowohl darum handeln, Ermüdungserscheinungen abzuhelfen, als auch einen frischen Duft zu vermitteln oder Hautrauhigkeiten zu vermeiden.

Es hat daher nicht an Bemühungen gefehlt, Fasern und daraus hergestellten Textilien mit kosmetischen Wirkstoffen auszurüsten, die beim Tragen auf die Haut übergehen und dort die gewünschten Effekte hervorrufen. Nun liegt es in der Natur der Sache, dass die gewünschten Wirkungen nur dann zustande kommen, wenn der entsprechende Wirkstoff vom Träger auf die Haut übertragen wird, d.h. nach einer mehr oder weniger langen Tragezeit ist auf dem Bekleidungsstück kein Wirkstoff mehr vorhanden. Dies stellt an den Hersteller solcher Produkte gewisse Anforderungen bei der Auswahl der Wirkstoffe, denn unter Abwägung von Leistung, aufbringbarer Menge und nicht zuletzt der damit verbundenen Kosten muss er einen Kompromiss finden, der ein Produkt ermöglicht, dessen Wirkung erlebbar ist und dessen erhöhter Preis auf vom Kunden gezahlt werden kann. Da kosmetische Wirkstoffe, die die gewünschten Wirkungen aufweisen, in aller Regel teuer sind und auch die Ausrüstung der Endprodukte mit zusätzlichen Kosten verbunden ist, ist es für den Hersteller von besonderer Bedeutung, dass es außer durch den Kontakt zwischen ausgerüstetem Endprodukt und der Haut des Trägers nicht zu weiteren unerwünschten Verlusten an Wirkstoffen kommt, da dies dazu führen würde, dass der vom Kunden teuer bezahlte zusätzliche Tragekomfort über eine kürzere Zeit wirksam wird.

Eine besonders unerwünschte Form des Wirkstoffverlustes tritt im Zusammenhang mit der Wäsche der so ausgerüsteten Fasern und Textilien auf. Auch wenn sich diese Verluste nicht völlig vermeiden lassen, so liegt es auf der Hand, dass es ein besonderes Anliegen der Hersteller entsprechender Produkte ist, die Wirkstoffe in solcher Weise auf die Fasern aufzubringen, dass diese nicht ohne weiteres aufgelöst oder mechanisch abgelöst werden.

Anstelle der vielfach durchführten Imprägnierverfahren, bei denen die Wirkstoffe direkt auf die Fasern oder Textilien aufgebracht werden, hat daher in den letzten Jahren der Einsatz von mikroverkapselten Wirkstoffen an Bedeutung gewonnen. Dahinter steht die Überlegung, wasserlösliche oder wasserdispergierbare Wirkstoffe in wasserunlöslichen Kapseln einzuschließen, welche die aktiven Prinzipien während des Tragens entweder durch kontrollierte Freisetzung durch Membranporen oder durch mechanische Zerstörung der Hüllmembranen abgeben. Auf diese Weise lassen sich die Verluste, die im Verlauf vieler Waschzyklen auftreten, im Vergleich zum Einsatz unverkapselter Wirkstoffe tatsächlich beträchtlich vermindern. Die so erzielten Ergebnisse sind in Summe jedoch längst nicht zufriedenstellend, da die verkapselten Wirkstoffen nur locker zwischen den Faserfibrillen gelagert sind und somit beispielsweise durch mechanische Einwirkung während des Waschvorgangs leicht ausgespült werden können. Ein ähnliches Problem trifft man im Bereich der Haarkosmetik an, wo es um die Haftung von verkapselten Wirkstoffen auf den natürlichen Keratinfasern geht.

Die Aufgabe der vorliegenden Erfindung hat folglich darin befanden, Wirkstoffzubereitungen für die Behandlung von natürlichen oder synthetischen Fasern zur Verfügung zu stellen, die frei von den oben geschilderten Nachteilen sind, d.h. die neben einer optimalen Avivage die vorteilhaften zusätzlichen Eigenschaften auch über eine Vielzahl von Waschzyklen aufweisen, ohne dass es zu nennenswerten Wirkstoffverlusten während der Wäsche kommt.

### Beschreibung der Erfindung

Gegenstand der Erfindung ist die Verwendung von Tensidzubereitungen, enthaltend
(a) kationische Tenside, und
(b) Mikrokapseln mit mittleren Durchmessern im Bereich von 0,0001 bis 5 mm, bestehend aus einer Hüllmembran und einer die Wirkstoffe enthaltenden Matrix, welche dadurch erhältlich sind, dass man
   (b11) aus Gelbildnern, Chitosanen und Wirkstoffen eine Matrix zubereitet,
   (b12) gegebenenfalls die Matrix in einer Ölphase dispergiert,
   (b13) die dispergierte Matrix mit wässrigen Lösungen anionischer Polymere behandelt und gegebenenfalls dabei die Ölphase entfernt.
   oder
   (b21) aus Gelbildnern, anionischen Polymeren und Wirkstoffen eine Matrix zubereitet,
   (b22) gegebenenfalls die Matrix in einer Ölphase dispergiert,
   (b23) die dispergierte Matrix mit wässrigen Chitosanlösungen behandelt und gegebenenfalls dabei die Ölphase entfernt.
   oder
   (b31) wässrige Wirkstoffzubereitungen mit Ölkörpern in Gegenwart von Emulgatoren zu O/W-Emulsionen verarbeitet,
   (b32) die so erhaltenen Emulsionen mit wässrigen Lösungen anionischer Polymere behandelt,
   (b33) die so erhaltene Matrix mit wässrigen Chitosanlösungen in Kontakt bringt und
   (b34) die so erhaltenen Verkapselungsprodukte von der wässrigen Phase abtrennt,
als Textilbehandlungsmittel.

Die erfindungsgemäß eingesetzten Zubereitungen verleihen sowohl synthetischen als auch natürlichen Fasern nicht nur einen angenehmen Weichgriff und verringern die elektrostatische Aufladung - was sich im Bereich der Textilien beispielsweise durch leichteres Bügeln, im Haarsektor durch verbesserte Trocken- und Nasskämmbarkeit bemerkbar macht - überraschenderweise wurde gefunden, dass Tenside, die eine Substantivität gegenüber Fasern aufweisen, speziell kationische Tenside und innerhalb dieser Gruppe insbesondere solche vom Esterquat-Typ, auch eine Substantivität gegenüber Mikrokapseln besitzen, speziell solchen, die Chitosan als Hüllsubstanz aufweisen. Durch die doppelte Substantivität vermitteln die Tenside den Mikrokapseln eine verbesserte Haftung, d.h. die mit der Freisetzung der darin enthaltenen Wirkstoffen verbundenen zusätzlichen Effekte, beispielsweise ein spezieller Duft, ein Frischegefühl, oder eine andere sensorische Wahrnehmung auf der Haut, werden vom Verbraucher über einen längeren Zeitraum, d.h. auch nach verschiedenen Waschzyklen wahrgenommen.

### Oberflächenaktive Verbindungen

Im Sinne der vorliegenden Erfindung werden als Komponente (a) kationische Tenside eingesetzt, darunter typische Tetraalkylammoniumverbindungen, wie beispielsweise Dimethyldistearylammoniumchlorid. Als besonders wirksam haben sich indes kationische Tenside vom Typ der Esterquats erwiesen.

### Esterquats

Unter der Bezeichnung "Esterquats" werden im allgemeinen quaternierte Fettsäuretriethanolaminestersalze verstanden. Es handelt sich dabei um bekannte Stoffe, die man nach den einschlägigen Methoden der präparativen organischen Chemie erhalten kann. Zu ihrer Herstellung geht man im allgemeinen von Fettsäuren aus, die man mit Triethanolamin oder Methyldiethanolamin in Gegenwart von unterphosphoriger Säure mit partiell verestert und anschließend in einer Lösung von Isopropylalkohol mit Dimethylsulfat oder Ethylenoxid quaterniert. Anstelle niederer Alkohole können auch Fettalkohole oder nichtionische Tenside als Emulgatoren bzw. Dispergatoren eingesetzt werden.

Die quaternierten Fettsäuretriethanolaminestersalze folgen in einer ersten Ausführungsform der Erfindung der Formel **(I),** 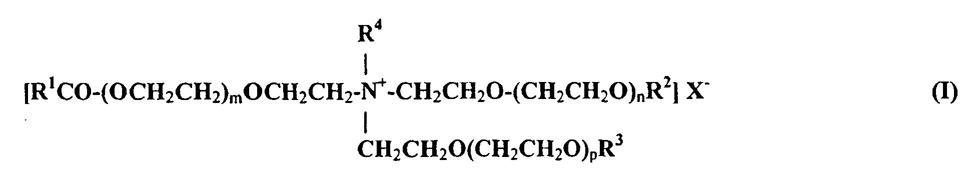 in der R¹CO für einen Acylrest mit 6 bis 22 Kohlenstoffatomen, R² und R³ unabhängig voneinander für Wasserstoff oder R¹CO, R⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht. Typische Beispiele für Esterquats, die im Sinne der Erfindung Verwendung finden können, sind Produkte auf Basis von Capronsäure, Caprylsäure, Caprinsäure, Laurinsäure, Myristinsäure, Palmitinsäure, Isostearinsäure, Stearinsäure, Ölsäure, Elaidinsäure, Arachinsäure, Behensäure und Erucasäure sowie deren technische Mischungen, wie sie beispielsweise bei der Druckspaltung natürlicher Fette und Öle anfallen. Vorzugsweise werden technische C_{12/18}-Kokosfettsäuren und insbesondere teilgehärtete C_{16/18}-Talg- bzw. Palmfettsäuren sowie elaidinsäurereiche C_{16/18}-Fettsäureschnitte eingesetzt. Zur Herstellung der quaternierten Ester können die Fettsäuren und das Triethanolamin im molaren Verhältnis von 1,1 : 1 bis 3 : 1 eingesetzt werden. Im Hinblick auf die anwendungstechnischen Eigenschaften der Esterquats hat sich ein Einsatzverhältnis von 1,2 : 1 bis 2,2 : 1, vorzugsweise 1,5 : 1 bis 1,9 : 1 als besonders vorteilhaft erwiesen. Die bevorzugten Esterquats stellen technische Mischungen von Mono-, Di- und Triestern mit einem durchschnittlichen Veresterungsgrad von 1,5 bis 1,9 dar und leiten sich von technischer C_{16/18}- Talg- bzw. Palmfettsäure (Iodzahl 0 bis 40) ab. Aus anwendungstechnischer Sicht haben sich quaternierte Fettsäuretriethanolaminestersalze der Formel (I) als besonders vorteilhaft erwiesen, in der R¹CO für einen Acylrest mit 16 bis 18 Kohlenstoffatomen, R² für R¹CO, R³ für Wasserstoff, R⁴ für eine Methylgruppe, m, n und p für 0 und X für Methylsulfat steht. Entsprechende Produkte sind unter der Marke Dehyquart® AU (Cognis Deutschland GmbH) im Handel.

Neben den quaternierten Fettsäuretriethanolaminestersalzen kommen als Esterquats ferner auch quaternierte Estersalze von Fettsäuren mit Diethanolalkylaminen der Formel **(II)** in Betracht, 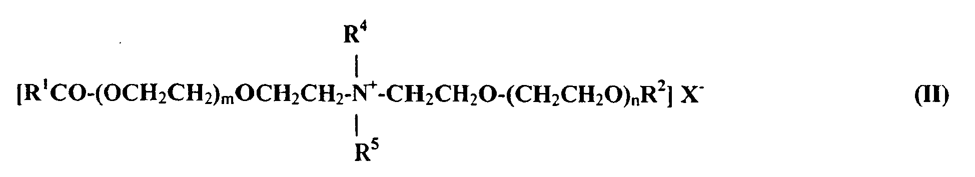 in der R¹CO für einen Acylrest mit 6 bis 22 Kohlenstoffatomen, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Als weitere Gruppe geeigneter Esterquats sind schließlich die quaternierten Estersalze von Fettsäuren mit 1,2-Dihydroxypropyldialkylaminen der Formel **(III)** zu nennen, 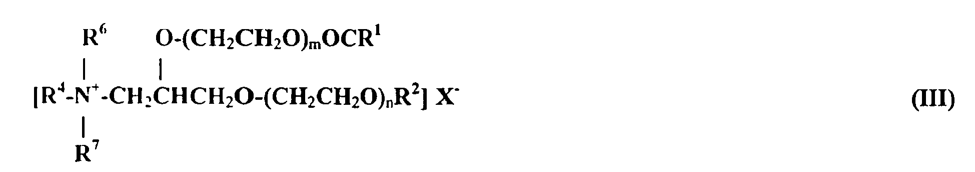 in der R¹CO für einen Acylrest mit 6 bis 22 Kohlenstoffatomen, R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Des weiteren kommen als Esterquats noch Stoffe in Frage, bei denen die Ester- durch eine Amidbindung ersetzt ist und die vorzugsweise basierend auf Diethylentriamin der Formel **(IV)** folgen, 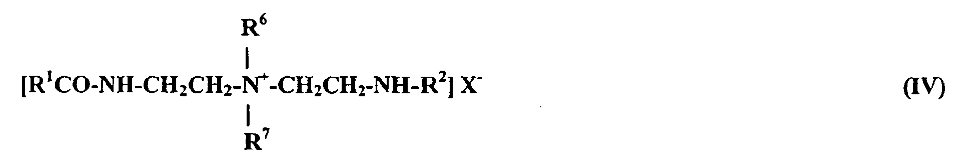 in der R¹CO für einen Acylrest mit 6 bis 22 Kohlenstoffatomen, R² für Wasserstoff oder R¹CO, R⁶ und R⁷ unabhängig voneinander für Alkylreste mit 1 bis 4 Kohlenstoffatomen und X für Halogenid, Alkylsulfat oder Alkylphosphat steht. Derartige Amidesterquats sind beispielsweise unter der Marke Incroquat® (Croda) im Markt erhältlich.

Schließlich kommen als Esterquats auch Stoffe in Frage, die auf Basis von ethoxyliertem Ricinusöl oder dessen Härtungsprodukten erhältlich sind und vorzugsweise der Formel **(V)** folgen, 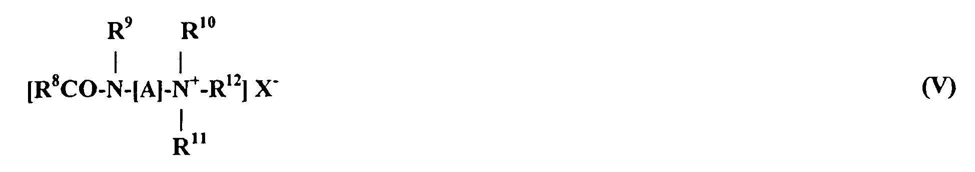 in der R⁸CO für einen gesättigten und/oder ungesättigten ethoxylierten Hydroxyacylrest mit 16 bis 22, vorzugsweise 18 Kohlenstoffatomen sowie 1 bis 50 Oxyethyleneinheiten, A für einen linearen oder verzweigten Alkylenrest mit 1 bis 6 Kohlenstoffatomen, R⁹, R¹⁰ und R¹¹ unabhängig voneinander für Wasserstoff oder eine Alkylgruppe mit 1 bis 4 Kohlenstoffatomen, R¹² für einen Alkylrest mit 1 bis 4 Kohlenstoffatomen oder einen Benzylrest und X für Halogen, Alkylsulfat oder Alkylphosphat steht.

Hinsichtlich der Auswahl der bevorzugten Fettsäuren und des optimalen Veresterungsgrades gelten die für **(I)** genannten Beispiele auch für die Esterquats der Formeln **(II)** bis **(V).**

Zur Herstellung der Esterquats der Formeln (I) bis (V) kann sowohl von Fettsäuren als auch den entsprechenden Triglyceriden ausgegangen werden. Ebenfalls ist es möglich, die Kondensation der Alkanolamine mit den Fettsäuren in Gegenwart definierter Mengen an Dicarbonsäuren, wie z.B. Oxalsäure, Malonsäure, Bernsteinsäure, Maleinsäure, Fumarsäure, Glutarsäure, Adipinsäure, Sorbinsäure, Pimelinsäure, Azelainsäure, Sebacinsäure und/oder Dodecandisäure durchzuführen. Auf diese Weise kommt es zur einer partiell oligomeren Struktur der Esterquats, was sich insbesondere bei Mitverwendung von Adipinsäure auf die Klarlöslichkeit der Produkte vorteilhaft auswirken kann. Entsprechende Produkte unter der Marke Dehyquart® D 6003 (Cognis) sind im Handel erhältlich. Üblicherweise gelangen die Esterquats in Form 50 bis 90 Gew.-%iger alkoholischer Lösungen in den Handel, die bei Bedarf problemlos mit Wasser verdünnt werden können.

### Wirkstoffe

Die Auswahl der Wirkstoffe ist an sich unkritisch und richtet sich ausschlich danach, welchen zusätzlichen Effekt neben dem Weichgriff das Endprodukt schließlich bewirken soll.

### ➢ Biogene Wirkstoffe

Bevorzugt sind solche biogenen Wirkstoffe, die feuchtigkeitsspendende Eigenschaften aufweisen, Cellulitis entgegenwirken und/oder selbstbräunend sind. Typische Beispiele sind Tocopherol, Tocopherolacetat, Tocopherolpalmitat, Carotine, Koffein, Ascorbinsäure, (Desoxy)Ribonukleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, Chitosan, Dihydroxyaceton, Menthol, Squalan, essentielle Öle (z.B. Jojobaöl), pflanzliche Proteine und deren Hydrolyseprodukte, Pflanzenextrakte, wie z.B. Prunusextrakt, Bambaranussextrakt und Vitaminkomplexe zu verstehen. Besonders bevorzugt ist der Einsatz von Squalan, Chitosan, Menthol, Retinol (Vitamin A), Koffein, pflanzlichen Proteinen und deren Hydrolyseprodukten, Carotinen und Jojobaöl, da diese zum Gleichgewicht der cutanen Hydrolipidschicht beitragen, dem Wasserverlust und damit der Faltenbildung vorbeugen, die Haut erfrischen und Ermüdungserscheinungen entgegenwirken, der Haut ein weiches und elastisches Gefühl verleihen, die Hautdrainage, die Zufuhr von Nährstoffen und die Blutzirkulation verbessern, gegen oxidativen Stress, Umweltgifte, Hautalterung und freie Radikale wirken, den durch Wasser und Sonne bewirkten Verlust an Fetten ausgleichen, die Wasserbeständigkeit von UV-Filtern verbessern, eine homogene Bräunung gewährleisten und schließlich zudem auch antimikrobielle Eigenschaften besitzen.

### ➢ Antioxidantien

Antioxidantien unterbrechen die photochemische Reaktionskette, welche ausgelöst wird, wenn UV-Strahlung in die Haut eindringt und sind daher als Wirkstoffe ebenfalls bevorzugt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. VitaminE-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### ➢ UV-Lichtschutzfilter

Zu den in verkapselter Form vorliegenden Wirkstoffen können ferner auch UV-Lichtschutzfilter zählen. Hierunter sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
➢ 3-Benzylidencampher bzw. 3-Benzylidennorcampher und dessen Derivate, z.B. 3-(4-Methylbenzyliden)campher beschrieben;
➢ 4-Aminobenzoesäurederivate, vorzugsweise 4-(Dimethylamino)benzoesäure-2-ethyl-hexylester, 4-(Dimethylamino)benzoesäure-2-octylester und 4-(Dimethylamino)benzoesäureamylester;
Ester der Zimtsäure, vorzugsweise 4-Methoxyzimtsäure-2-ethylhexylester, 4-Methoxy-zimtsäurepropylester, 4-Methoxyzimtsäureisoamylester 2-Cyano-3,3-phenylzimtsäure-2-ethylhexylester (Octocrylene);
➢ Ester der Salicylsäure, vorzugsweise Salicylsäure-2-ethylhexylester, Salicylsäure-4-iso-propylbenzylester, Salicylsäurehomomenthylester;
➢ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
➢ Ester der Benzalmalonsäure, vorzugsweise 4-Methoxybenzmalonsäuredi-2-ethylhexylester;
➢ Triazinderivate, wie z.B. 2,4,6-Trianilino-(p-carbo-2'-ethyl-1'-hexyloxy)-1,3,5-triazin und Octyl Triazon oder Dioctyl Butamido Triazone (Uvasorb® HEB);
➢ Propan-1,3-dione, wie z.B. 1-(4-tert.Butylphenyl)-3-(4'methoxyphenyl)propan-1,3-dion;
➢ Ketotricyclo(5.2.1.0)decan-Derivate.

Als wasserlösliche Substanzen kommen in Frage:
➢ 2-Phenylbenzimidazol-5-sulfonsäure und deren Alkali-, Erdalkali-, Ammonium-, Alkylammonium-, Alkanolammonium- und Glucammoniumsalze;
➢ Sulfonsäurederivate von Benzophenonen, vorzugsweise 2-Hydroxy-4-methoxybenzophenon-5-sulfonsäure und ihre Salze;
➢ Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 1-Phenyl-3-(4'-isopropylphenyl)-propan-1,3-dion sowie Enaminverbindungen. Die UV-A und UV-B-Filter können selbstverständlich auch in Mischungen eingesetzt werden. Besonders günstige Kombinationen bestehen aus den Derivate des Benzoylmethans,, z.B. 4-tert.-Butyl-4' methoxydibenzoylmethan (Parsol® 1789) und 2-Cyano-3,3-phenylzimtsäure-2-ethylhexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden deartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet.

### ➢ Deodorantien

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren und ebenfalls als zu verkapselnde Wirkstoffe von Interesse sind.

Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxy-diphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-Iod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Famesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen.

Antitranspirantien (Antiperspirantien) reduzieren durch Beeinflussung der Aktivität der ekkrinen Schweißdrüsen die Schweißbildung, und wirken somit Achselnässe und Körpergeruch entgegen. Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin.

### ➢ Parfümöle und Aromen

Parfümöle und Aromen stellen eine weitere Gruppe von Wirkstoffen dar, die in verkapselter Form Bestandteil der erfindungsgemäßen Zubereitungen sein können. Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Y-lang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt. Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

Der Anteil der Wirkstoffe an den Mikrokapseln kann 1 bis 30, vorzugsweise 5 bis 25 und insbesondere 15 bis 20 Gew.-% betragen.

### Mikrokapseln

Unter dem Begriff "Mikrokapsel" werden vom Fachmann sphärische Aggregate mit einem Durchmesser im Bereich von etwa 0,0001 bis etwa 5 mm verstanden, die mindestens einen festen oder flüssigen Kern enthalten, der von mindestens einer kontinuierlichen Hülle umschlossen ist. Genauer gesagt handelt es sich um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Nach einem anderen Verfahren werden geschmolzene Wachse in einer Matrix aufgenommen ("microsponge"), die als Mikropartikel zusätzlich mit filmbildenden Polymeren umhüllt sein können. Die mikroskopisch kleinen Kapseln, auch Nanokapseln genannt, lassen sich wie Pulver trocknen. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccaride, wie Stärke oder Dextran, Polypeptide, Proteinghydrolysate, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon.

Beispiele für Mikrokapseln des Stands der Technik sind folgende Handelsprodukte (in Klammern angegeben ist jeweils das Hüllmaterial) : *Hallcrest Microcapsules* (Gelatine, Gummi Arabicum), *Coletica Thalaspheres* (maritimes Collagen), *Lipotec Millicapseln* (Alginsäure, Agar-Agar), *Induchem Unispheres* (Lactose, mikrokristalline Cellulose, Hydroxypropylmethylcellulose); *Unicerin C30* (Lactose, mikrokristalline Cellulose, Hydroxypropylmethylcellulose), *Kobo Glycospheres* (modifizierte Stärke, Fettsäureester, Phospholipide), *Softspheres* (modifiziertes Agar-Agar) und *Kuhs Probiol Nanospheres* (Phospholipide) sowie *Primaspheres* und *Primasponges* (Chitosan, Alginate) und *Primasys* (Phospholipide).

Chitosanmikrokapseln und Verfahren zu ihrer Herstellung sind Gegenstand früherer Patenanmeldungen der Patentanmelderin **[**WO 01/01926**,** WO 01/01927**,** WO 01/01928**,** WO 01/01929]. Die gemäß Anspruch 1 einzusetzenden Mikrokapseln mit mittleren Durchmessern im Bereich von 0,0001 bis 5, vorzugsweise 0,001 bis 0,5 und insbesondere 0,005 bis 0,1 mm, bestehend aus einer Hüllmembran und einer die Wirkstoffe enthaltenden Matrix, werden erhalten, indem man
(a1) aus Gelbildnern, Chitosanen und Wirkstoffen eine Matrix zubereitet,
(a2) gegebenenfalls die Matrix in einer Ölphase dispergiert,
(a3) die dispergierte Matrix mit wässrigen Lösungen anionischer Polymere behandelt und gegebenenfalls dabei die Ölphase entfernt.
oder
(b1) aus Gelbildnern, anionischen Polymeren und Wirkstoffen eine Matrix zubereitet,
(b2) gegebenenfalls die Matrix in einer Ölphase dispergiert,
(b3) die dispergierte Matrix mit wässrigen Chitosanlösungen behandelt und gegebenenfalls dabei die Ölphase entfernt.
oder
(c1) wässrige Wirkstoffzubereitungen mit Ölkörpern in Gegenwart von Emulgatoren zu O/W-Emulsionen verarbeitet,
(c2) die so erhaltenen Emulsionen mit wässrigen Lösungen anionischer Polymere behandelt,
(c3) die so erhaltene Matrix mit wässrigen Chitosanlösungen in Kontakt bringt und
(c4) die so erhaltenen Verkapselungsprodukte von der wässrigen Phase abtrennt.

### Gelbildner

Im Sinne der Erfindung werden als Gelbildner vorzugsweise solche Stoffe in Betracht gezogen, welche die Eigenschaft zeigen in wässriger Lösung bei Temperaturen oberhalb von 40 °C Gele zu bilden. Typische Beispiele hierfür sind Heteropolysaccharide und Proteine. Als thermogelierende Heteropolysaccharide kommen vorzugsweise Agarosen in Frage, welche in Form des aus Rotalgen zu gewinnenden Agar-Agar auch zusammen mit bis zu 30 Gew.-% nicht-gelbildenden Agaropektinen vorliegen können. Hauptbestandteil der Agarosen sind lineare Polysaccharide aus D-Galaktose und 3,6-Anhydro-L-galaktose, die alternierend β-1,3-und β-1,4-glykosidisch verknüpft sind. Die Heteropolysaccharide besitzen vorzugsweise ein Molekulargewicht im Bereich von 110.000 bis 160.000 und sind sowohl farb- als auch geschmacklos. Als Alternativen kommen Pektine, Xanthane (auch Xanthan Gum) sowie deren Mischungen in Frage. Es sind weiterhin solche Typen bevorzugt, die noch in 1-Gew.-%iger wässriger Lösung Gele bilden, die nicht unterhalb von 80 °C schmelzen und sich bereits oberhalb von 40 °C wieder verfestigen. Aus der Gruppe der thermogelierenden Proteine seien exemplarisch die verschiedenen Gelatine-Typen genannt.

### Chitosane

Chitosane stellen Biopolymere dar und werden zur Gruppe der Hydrokolloide gezählt. Chemisch betrachtet handelt es sich um partiell deacetylierte Chitine unterschiedlichen Molekulargewichtes, die den folgenden - idealisierten - Monomerbaustein enthalten:

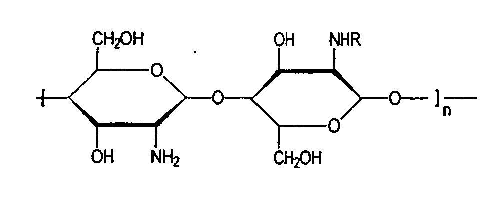

Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in Wechselwirkung treten und werden daher in kosmetischen Haar- und Körperpflegemitteln sowie pharmazeutischen Zubereitungen eingesetzt. Zur Herstellung der Chitosane geht man von Chitin, vorzugsweise den Schalenresten von Krustentieren aus, die als billige Rohstoffe in großen Mengen zur Verfügung stehen. Das Chitin wird dabei in einem Verfahren, das erstmals von Hackmann et al. beschrieben worden ist, üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Vorzugsweise werden solche Typen eingesetzt, wie die ein durchschnittliches Molekulargewicht von 10.000 bis 500.000 bzw. 800.000 bis 1.200.000 Dalton aufweisen und/oder eine Viskosität nach Brookfield (1 Gew.-%ig in Glycolsäure) unterhalb von 5000 mPas, einen Deacetylierungsgrad im Bereich von 80 bis 88 % und einem Aschegehalt von weniger als 0,3 Gew.-% besitzen. Aus Gründen der besseren Wasserlöslichkeit werden die Chitosane in der Regel in Form ihrer Salze, vorzugsweise als Glycolate eingesetzt.

### Ölphase

Die Matrix kann vor der Bildung der Membran optional in einer Ölphase dispergiert werden. Als Öle kommen für diesen Zweck beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Öleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglycerid-mischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestern mit Polyolen, Siliconöle und/oder aliphatische bzw. naphthenische Kohlenwasserstoffe, wie z.B. wie Squalan, Squalen oder Dialkylcyclohexane in Betracht.

### Anionpolymere

Die anionische Polymere haben die Aufgabe, mit den Chitosanen Membranen zu bilden. Für diesen Zweck eignen sich vorzugsweise Salze der Alginsäure. Bei der Alginsäure handelt es sich um ein Gemisch carboxylgruppenhaltiger Polysaccharide mit folgendem idealisierten Monomerbaustein:

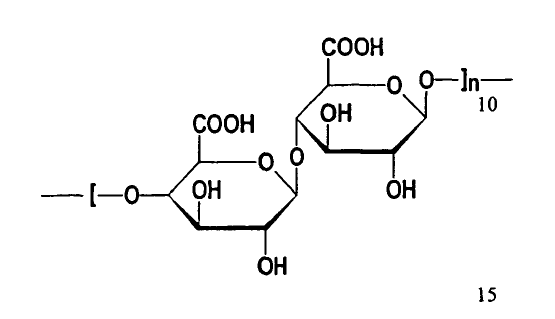

Das durchschnittliche Molekulargewicht der Alginsäuren bzw. der Alginate liegt im Bereich von 150.000 bis 250.000. Dabei sind als Salze der Alginsäure sowohl deren vollständige als auch deren partiellen Neutralisationsprodukte zu verstehen, insbesondere die Alkalisalze und hierunter vorzugsweise das Natriumalginat ("Algin") sowie die Ammonium- und Erdalkalisalze. besonders bevorzugt sind Mischalginate, wie z.B. Natrium/Magnesium- oder Natrium/Calciumalginate. In einer alternativen Ausführungsform der Erfindung kommen für diesen Zweck jedoch auch anionische Chitosanderivate, wie z.B. Carboxylierungs- und vor allem Succinylierungsprodukte in Frage. Alternativ kommen auch Poly(meth)acrylate mit durchschnittlichen Molekulargewichten im Bereich von 5.000 bis 50.000 Dalton sowie die verschiedenen Carboxymethylcellulosen in Frage. Anstelle der anionischen Polymeren können für die Ausbildung der Hüllmembran auch anionische Tenside oder niedermolekulare anorganische Salze, wie beispielsweise Pyrophosphate eingesetzt werden.

### Emulgatoren

Als Emulgatoren kommen beispielsweise nichtionogene Tenside aus mindestens einer der folgenden Gruppen in Frage:
➢ Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
➢ Alkyl- und/oder Alkenyloligoglykoside mit 8 bis 22 Kohlenstoffatomen im Alk(en)ylrest und deren ethoxylierte Analoga;
➢ Anlagerungsprodukte von 1 bis 15 Mol Ethylenoxid an Ricinusöl und/oder gehärtetes Ricinusöl;
➢ Anlagerungsprodukte von 15 bis 60 Mol Ethylenoxid an Ricinusöl und/oder gehärtetes Ricinusöl;
➢ Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
➢ Mono-, Di- und Trialkylphosphate sowie Mono-, Di- und/oder Tri-PEG-alkylphosphate und deren Salze;
➢ Wollwachsalkohole;
➢ Polysiloxan-Polyalkyl-Polyether-Copolymere bzw. entsprechende Derivate;
➢ Block-Copolymere z.B. Polyethylenglycol-30 Dipolyhydroxystearate;
➢ Polymeremulgatoren, z.B. Pemulen-Typen (TR-1,TR-2) von Goodrich;
➢ Polyalkylenglycole sowie
➢ Glycerincarbonat.

### ➢ Ethylenoxidanlagerungsprodukte

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind als Rückfettungsmittel für kosmetische Zubereitungen bekannt.

### ➢ Alkyl- und/oder Alkenyloligoglykoside

Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

### ➢ Partialglyceride

Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

### ➢ Sorbitanester

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesqui-tartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

### ➢ Polyglycerinester

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

### ➢ Anionische Emulgatoren

Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.

### ➢ Amphotere und kationische Emulgatoren

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine - COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe.. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

### Herstellverfahren Mikrokapseln

Zur Herstellung der Mikrokapseln stellt man üblicherweise eine 1 bis 10, vorzugsweise 2 bis 5 Gew.-%ige wässrige Lösung des Gelbildners, vorzugsweise des Agar-Agars her und erhitzt diese unter Rückfluss. In der Siedehitze, vorzugsweise bei 80 bis 100°C, wird eine zweite wässrige Lösung zugegeben, welche das Chitosan in Mengen von 0,1 bis 2, vorzugsweise 0,25 bis 0,5 Gew.-% und den Wirkstoff (Komponenten a und b) in Mengen von 0,1 bis 25 und insbesondere 0,25 bis 10 Gew.-% enthält; diese Mischung wird als Matrix bezeichnet. Die Beladung der Mikrokapseln mit Wirkstoffen kann daher ebenfalls 0,1 bis 25 Gew.-% bezogen auf das Kapselgewicht betragen. Falls gewünscht, können zu diesem Zeitpunkt zur Viskositätseinstellung auch wasserunlösliche Bestandteile, beispielsweise anorganische Pigmente zugegeben werden, wobei man diese in der Regel in Form von wässrigen oder wässrig/alkoholischen Dispersionen zusetzt. Zur Emulgierung bzw. Dispergierung der Wirkstoffe kann es ferner von Nutzen sein, der Matrix Emulgatoren und/oder Lösungsvermittler hinzuzugeben. Nach der Herstellung der Matrix aus Gelbildner, Chitosan und Wirkstoffmischung (Komponenten a und b) kann die Matrix optional in einer Ölphase unter starker Scherung sehr fein dispergiert werden, um bei der nachfolgenden Verkapselung möglichst kleine Teilchen herzustellen. Dabei hat es sich als besonders vorteilhaft erwiesen, die Matrix auf Temperaturen im Bereich von 40 bis 60 °C zu erwärmen, während man die Ölphase auf 10 bis 20 °C kühlt. Im letzten, nun wieder obligatorischen Schritt erfolgt dann die eigentliche Verkapselung, d.h. die Ausbildung der Hüllmembran durch Inkontaktbringen des Chitosans in der Matrix mit den anionischen Polymeren. Hierzu empfiehlt es sich, die gegebenenfalls in der Ölphase dispergierte Matrix bei einer Temperatur im Bereich von 40 bis 100, vorzugsweise 50 bis 60 °C mit einer wässrigen, etwa 1 bis 50 und vorzugsweise 10 bis 15 Gew.-%ige wässrigen Lösung des Anionpolymers zu behandeln und dabei - falls erforderlich - gleichzeitig oder nachträglich die Ölphase zu entfernen. Die dabei resultierenden wässrigen Zubereitungen weisen in der Regel einen Mikrokapselgehalt im Bereich von 1 bis 10 Gew.-% auf. In manchen Fällen kann es dabei von Vorteil sein, wenn die Lösung der Polymeren weitere Inhaltsstoffe, beispielsweise Emulgatoren oder Konservierungsmittel enthält. Nach Filtration werden Mikrokapseln erhalten, welche im Mittel einen Durchmesser im Bereich von vorzugsweise etwa 1 mm aufweisen. Es empfiehlt sich, die Kapseln zu sieben, um eine möglichst gleichmäßige Größenverteilung sicherzustellen. Die so erhaltenen Mikrokapseln können im herstellungsbedingten Rahmen eine beliebige Form aufweisen, sie sind jedoch bevorzugt näherungsweise kugelförmig. Alternativ kann man die Anionpolymere auch zur Herstellung der Matrix einsetzen und die Verkapselung mit den Chitosanen durchführen.

In einem alternativen Verfahren wird zur Herstellung der erfindungsgemäßen Mikrokapseln wird zunächst eine O/W-Emulsion zubereitet, welche neben dem Ölkörper, Wasser und dem Wirkstoff eine wirksame Menge Emulgator enthält. Zur Herstellung der Matrix wird diese Zubereitung unter starkem Rühren mit einer entsprechenden Menge einer wäßrigen Anionpolymerlösung versetzt. Die Membranbildung erfolgt durch Zugabe der Chitosanlösung. Der gesamte Vorgang findet vorzugsweise im schwach sauren Bereich bei pH = 3 bis 4 statt. Falls erforderlich erfolgt die pH-Einstellung durch Zugabe von Mineralsäure. Nach der Membranbildung wird der pH-Wert auf 5 bis 6 angehoben, beispielsweise durch Zugabe von Triethanolamin oder einer anderen Base. Hierbei kommt es zu einem Anstieg der Viskosität, die durch Zugabe von weiteren Verdickungsmitteln, wie z.B. Polysacchariden, insbesondere Xanthan-Gum, Guar-Guar, Agar-Agar, Alginaten und Tylosen, Carboxymethylcellulose und Hydroxyethylcellulose, höhermolekularen Polyethylenglycolmono- und -diesten von Fettsäuren, Polyacrylaten, Polyacrylamiden und dergleichen noch unterstützt werden kann. Abschließend werden die Mikrokapseln von der wässrigen Phase beispielsweise durch Dekantieren, Filtrieren oder Zentrifugieren abgetrennt.

### Konzentrierte Tensidzubereitungen und deren Verdünnungen

Bei den einzusetzenden Tensidzubereitungen kann es sich um Konzentrate handeln, die vom Hersteller der Endprodukte auf eine niedrigere Anwendungskonzentration verdünnt werden können. Typischerweise enthalten die Konzentrate
(a) 10 bis 90, vorzugsweise 30 bis 60 und ins besondere 40 bis 50 Gew.-% oberflächenaktive Substanzen, die in der Lage sind, natürlichen oder synthetischen Fasern einen angenehmen Weichgriff zu verleihen, und
(b) 1 bis 30, vorzugsweise 5 bis 25 und insbesondere 10 bis 20 Gew.-% mikroverkapselte Wirkstoffe
mit der Maßgabe, dass sich die Mengenangaben gegebenenfalls mit Wasser oder niederen aliphatischen Alkoholen oder Polyalkylenglycolen (wie beispielsweise Ethanol, Isopropylalkohol oder Propylenglycol) und polymeren Verdickungsmitteln zu 100 Gew.-% ergänzen.

Bei den polymeren Verdickungsmitteln handelt es sich um eine optionale Komponente, die den Konzentraten zugesetzt werden kann, wenn eine möglichst homogene und stabile Verteilung der Mikrokapseln in den Konzentraten erwünscht ist. Die Polymeren bauen ein dreidimensionales Netz und damit eine hinreichend hohe innerhalb der Lösung auf, in dem die Mikrokapseln dispergiert vorliegen. Typische Polymere, die für diesen Zweck in Frage kommen, sind Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone® Gel VS-5PC (Rheox) erwiesen, bei dem es sich um eine Mischung aus Cyclopentasiloxan, Disteardimonium Hectorit und Propylencarbonat handelt. Die gleichen Stoffen können auch in den Anwendungsverdünnungen verwendet werden. Ihre Einsatzmenge liegt in der Regel im Bereich von 1 bis 5 Gew.-% - bezogen auf die jeweiligen Zubereitungen.

Ein typisches Konzentrat setzt sich beispielsweise aus 60 Gew.-% Esterquat, 29 Gew.-% Mikrokapseln, 1 Gew.-% Polyacrylat und 10 Gew.-% Isopropylalkohol zusammen. Aus derartigen Konzentraten können im Anschluss Endprodukte, wie beispielsweise Textil behandlungsmittel hergestellt werden. Im einfachsten Fall stellen diese Mittel, also z.B. Wäscheweichspülmittel, wässrige Verdünnungen der Konzentrate dar, andernfalls können sie auch weitere typische Hilfs- und Zusatzstoffe aufweisen, bei deren Auswahl jeweils auf den speziellen Anwendungszweck abzustellen ist.

### Gewerbliche Anwendbarkeit

Die erfindungsgemäß eingesetzten Zubereitungen ziehen rasch auf synthetische wie auch natürliche (Keratin-)fasem auf und verleihen ihnen einen angenehmen Weichgriff. Durch die Substantitivität der weichmachenden oberflächenaktiven Stoffe, speziell der Kationtenside und insbesondere der Esterquats sowohl gegenüber den Fasern als auch den Mikrokapseln, wird eine verbesserte Haftung der Kapseln auf den fasern erreicht, d.h. der durch die verkapselten Wirkstoffe hervorgerufene zusätzliche Effekt kann vom Verbraucher über einen längeren Zeitraum wahrgenommen werden.

### Beispiele

**Herstellbeispiel H1.** In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Talk in ad 100 g Wasser und dann mit einer Zubereitung von 25 g Chitosan (Hydagen® DCMF, 1 Gew.-%ig in Glycolsäure, Cognis, Düsseldorf/FRG), 5 g Menthol, 0,5 g Phenonip® (Konservierungsmittelmischung enthaltend Phenoxyethanol und Parabene) und 0,5 g Polysorbat-20 (Tween® 20, ICI) in ad 100 g Wasser versetzt. Die erhaltene Matrix wurde filtriert, auf 60 °C erwärmt und in eine 0,5 Gew.-%ige Natriumalginatlösung getropft. Nach dem Sieben wurde eine wässrige Zubereitung erhalten, die 8 Gew.-% Mikrokapseln mit einem mittleren Durchmesser von 1 mm enthielt. Anschließend wurden 29 g der Mikrokapseln unter Zusatz von 1 g Carbopol in 70 g einer Esterquatzubereitung vom Typ Dehyquart L® 80 (Dicocoylmethylethoxymonium Methosulfate (and) Propylenglycol, Cognis) dispergiert.

**Herstellbeispiel H2.** In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Talk in ad 100 g Wasser und dann mit einer Zubereitung von 25 g Chitosan (Hydagen® DCMF, 1 Gew.-%ig in Glycolsäure, Cognis, Düsseldorf/FRG), 5 g Tocopherol, 0,5 g Phenonip® (Konservierungsmittelmischung enthaltend Phenoxyethanol und Parabene) und 0,5 g Polysorbat-20 (Tween® 20, ICI) in ad 100 g Wasser versetzt. Die erhaltene Matrix wurde filtriert, auf 50 °C temperiert und unter starken Rühren im 2,5fachen Volumen Paraffinöl, das zuvor auf 15 °C gekühlt worden war, dispergiert. Die Dispersion wurde anschließend mit einer wässrigen Lösung enthaltend 1 Gew.-% Natriumlaurylsulfat und 0,5 Gew.-% Natriumalginat und dann mehrfach mit einer 0,5 Gew.-%igen wässrigen Phenoniplösung gewaschen, wobei die Ölphase entfernt wurde. Nach dem Sieben wurde eine wässrige Zubereitung erhalten, die 8 Gew.-% Mikrokapseln mit einem mittleren Durchmesser von 1 mm enthielt. Anschließend wurden 29 g der Mikrokapseln unter Zusatz von 1 g Carbopol in 70 g einer Esterquatzubereitung vom Typ Dehyquart L® 80 (Dicocoylmethylethoxymonium Methosulfate (and) Propylenglycol, Cognis) dispergiert.

**Herstellbeispiel H3.** In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Talk in ad 100 g Wasser und dann mit einer Zubereitung von 25 g Chitosan (Hydagen® DCMF, 1 Gew.-%ig in Glycolsäure, Cognis, Düsseldorf/FRG), 5 g Coffein, 0,5 g Phenonip® (Konservierungsmittelmischung enthaltend Phenoxyethanol und Parabene) und 0,5 g Polysorbat-20 (Tween® 20, ICI) in ad 100 g Wasser versetzt. Die erhaltene Matrix wurde filtriert, auf 60 °C erwärmt und in eine 15 Gew.-%ige Lösung von Sodium Laureth Sulfate getropft. Nach dem Sieben wurde eine wässrige Zubereitung erhalten, die 9 Gew.-% Mikrokapseln mit einem mittleren Durchmesser von 1 mm enthielt. Anschließend wurden 29 g der Mikrokapseln unter Zusatz von 1 g Carbopol in 70 g einer Esterquatzubereitung vom Typ Dehyquart L® 80 (Dicocoylmethylethoxymonium Methosulfate (and) Propylenglycol, Cognis) dispergiert.

**Herstellbeispiel H4.** In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Talk in ad 100 g Wasser und dann mit einer Zubereitung von 25 g Chitosan (Hydagen® DCMF, 1 Gew.-%ig in Glycolsäure, Cognis, Düsseldorf/FRG), 5 g Menthol, 0,5 g Phenonip® (Konservierungsmittelmischung enthaltend Phenoxyethanol und Parabene) und 0,5 g Polysorbat-20 (Tween® 20, ICI) in ad 100 g Wasser versetzt. Die erhaltene Matrix wurde filtriert, auf 60 °C erwärmt und in eine 15 Gew.-%ige Lösung von Natriumpyrophosphat getropft. Nach dem Sieben wurde eine wässrige Zubereitung erhalten, die 8 Gew.-% Mikrokapseln mit einem mittleren Durchmesser von 1 mm enthielt. Anschließend wurden 29 g der Mikrokapseln unter Zusatz von 1 g Carbopol in 70 g einer Esterquatzubereitung vom Typ Dehyquart L® 80 (Dicocoylmethylethoxymonium Methosulfate (and) Propylenglycol, Cognis) dispergiert.

**Herstellbeispiel H5.** In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Talk in ad 100 g Wasser und dann mit einer Zubereitung von 25 g Chitosan (Hydagen® DCMF, 1 Gew.-%ig in Glycolsäure, Cognis, Düsseldorf/FRG), 5 g Betacarotin, 0,5 g Phenonip® (Konservierungsmittelmischung enthaltend Phenoxyethanol und Parabene) und 0,5 g Polysorbat-20 (Tween® 20, ICI) in ad 100 g Wasser versetzt. Die so erhaltene Matrix wurde filtriert, auf 50 °C temperiert und unter starken Rühren im 2,5fachen Volumen Paraffinöl, das zuvor auf 15 °C gekühlt worden war, dispergiert. Die Dispersion wurde anschließend mit einer 15 Gew.-%igen Natriumpyrophosphatlösung und dann mehrfach mit einer 0,5 Gew.-%igen wässrigen Phenoniplösung gewaschen, wobei die Ölphase entfernt wurde. Nach dem Sieben wurde eine wässrige Zubereitung erhalten, die 10 Gew.-% Mikrokapseln mit einem mittleren Durchmesser von 1 mm enthielt. Anschließend wurden 29 g der Mikrokapseln unter Zusatz von 1 g Carbopol in 70 g einer Esterquatzubereitung vom Typ Dehyquart L® 80 (Dicocoylmethylethoxymonium Methosulfate (and) Propylenglycol, Cognis) dispergiert.

**Herstellbeispiel H6.** In einem 500-ml-Dreihalskolben mit Rührer und Rückflusskühler wurden in der Siedehitze 3 g Gelatine in 200 ml Wasser gelöst. Anschließend wurde die Mischung innerhalb von etwa 30 min unter starkem Rühren zunächst mit einer homogenen Dispersionen von 10 g Glycerin und 2 g Talk in ad 100 g Wasser und dann mit einer Zubereitung von 25 g Chitosan (Hydagen® DCMF, 1 Gew.-%ig in Glycolsäure, Henkel KGaA, Düsseldorf/FRG), 5 g Sojaprotein, 0,5 g Phenonip® in ad 100 g Wasser versetzt. Die erhaltene Matrix wurde filtriert, auf 60 °C erwärmt und in eine 0,5 Gew.-%ige Lösung von Hydagen® SCD (succinyliertes Chitosan, Cognis) getropft. Nach dem Sieben wurde eine wässrige Zubereitung erhalten, die 8 Gew.-% Mikrokapseln mit einem mittleren Durchmesser von 1 mm enthielt. Anschließend wurden 29 g der Mikrokapseln unter Zusatz von 1 g Carbopol in 70 g einer Esterquatzubereitung vom Typ Dehyquart L® 80 (Dicocoylmethylethoxymonium Methosulfate (and) Propylenglycol, Cognis) dispergiert.

**Herstellbeispiel H7.** In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Talk in ad 100 g Wasser und dann mit einer Zubereitung von 25 g Chitosan (Hydagen® DCMF, 1 Gew.-%ig in Glycolsäure, Cognis, Düsseldorf/FRG), 5 g Jojobaöl, 0,5 g Phenonip® (Konservierungsmittelmischung enthaltend Phenoxyethanol und Parabene) und 0,5 g Polysorbat-20 (Tween® 20, ICI) in ad 100 g Wasser versetzt. Die erhaltene Matrix wurde filtriert, auf 60 °C erwärmt und in eine 0,5 Gew.-%ige Natriumalginatlösung getropft. Zum Erhalt von Mikrokapseln gleichen Durchmessers wurden die Zubereitungen anschließend gesiebt. Anschließend wurden 29 g der Mikrokapseln unter Zusatz von 1 g Carbopol in 70 g einer Esterquatzubereitung vom Typ Dehyquart L® 80 (Dicocoylmethylethoxymonium Methosulfate (and) Propylenglycol, Cognis) dispergiert.

**Beispiel H8.** In einer Rührapparatur wurden 0,5 g Konservierungsmittel (Phenonip®) in 50 g einer 2 Gew.-%igen wässrigen Zubereitung von Carboxymethylcellulose gelöst und die Mischung auf pH = 3,5 eingestellt. Anschließend wurde unter starkem Rühren eine Mischung bestehend aus 10 g einer 10 Gew.-%igen Lösung von Ambroxan und 0,5 g Sorbitanmonostearat+20EO (Eumulgin® SMS 20, Cognis Deutschland GmbH) hinzugegeben. Danach wurde unter weiterem Rühren eine solche Menge einer 1 Gew.-%igen Lösung von Chitosan in Glycolsäure (Hydagen® CMF Cognis Deutschland GmbH) hinzugegeben, dass sich eine Chitosankonzentration von 0,075 Gew.-% - bezogen auf die Zubereitung - einstellte. Anschließend wurde der pH-Wert durch Zugabe von Triethanolamin auf 5,5 angehoben und die entstandenen Mikrokapseln dekantiert. Zum Abschluss wurden 29 g der Mikrokapseln unter Zusatz von 1 g Carbopol in 70 g einer Esterquatzubereitung vom Typ Dehyquart L® 80 (Dicocoylmethylethoxymonium Methosulfate (and) Propylenglycol, Cognis) dispergiert.

**Beispiel H9.** In einer Rührapparatur wurden 0,5 g Konservierungsmittel (Phenonip®) in 50 g einer 2 Gew.-%igen wässrigen Zubereitung von Polyacrylsäure (Pemulen® TR-2) gelöst, wobei sich ein pH-Wert von 3 einstellte. Anschließend wurde unter starkem Rühren eine Mischung bestehend aus 10 g einer 10 Gew.-%igen Lösung von Tocopherol und 0,5 g Sorbitanmonolaurat+15EO (Eumulgin® SML 15, Cognis Deutschland GmbH) hinzugegeben. Danach wurde unter weiterem Rühren eine solche Menge einer 1 Gew.-%igen Lösung von Chitosan in Glycolsäure (Hydagen® CMF Cognis Deutschland GmbH) hinzugegeben, dass sich eine Chitosankonzentration von 0,01 Gew.-% - bezogen auf die Zubereitung - einstellte. Anschließend wurde der pH-Wert durch Zugabe von Triethanolamin auf 5,5 angehoben und die entstandenen Mikrokapseln dekantiert. Zum Abschluss wurden 29 g der Mikrokapseln unter Zusatz von 1 g Carbopol in 70 g einer Esterquatzubereitung vom Typ Dehyquart L® 80 (Dicocoylmethylethoxymonium Methosulfate (and) Propylenglycol, Cognis) dispergiert.

**Beispiel H10.** In einer Rührapparatur wurden 0,5 g Konservierungsmittel (Phenonip®) in 50 g einer 2 Gew.-%igen wäßrigen Zubereitung von Polyacrylsäure (Pemulen® TR-2) gelöst, wobei sich ein pH-Wert von 3 einstellte. Anschließend wurde unter starkem Rühren eine Mischung bestehend aus 5 g Lösung von Koffein und 0,5 g Coco Glucosides (Plantacare APG 1200, Cognis Deutschland GmbH) hinzugegeben. Danach wurde unter weiterem Rühren eine solche Menge einer 1 Gew.-%igen Lösung von Chitosan in Glycolsäure (Hydagen® CMF Cognis Deutschland GmbH) hinzugegeben, dass sich eine Chitosankonzentration von 0,01 Gew.-% - bezogen auf die Zubereitung - einstellte. Anschließend wurde der pH-Wert durch Zugabe von Triethanolamin auf 5,5 angehoben und die entstandenen Mikrokapseln dekantiert. Zum Abschluss wurden 29 g der Mikrokapseln unter Zusatz von 1 g Carbopol in 70 g einer Esterquatzubereitung vom Typ Dehyquart L® 80 (Dicocoylmethylethoxymonium Methosulfate (and) Propylenglycol, Cognis) dispergiert.

**Anwendungsbeispiele.** Marktübliche Strumpfhosen wurden in der Waschmaschine dreimal mit einer Tensidzubereitung gemäß Herstellbeispiel H1 (30 min, 20 °C, 1 g/L Tensidzubereitung) behandelt und jeweils wieder getrocknet. Anschließend wurden die Strumpfhosen 30mal (a) in der Waschmaschine (30 min, 20 °C, 1 g/L Feinwaschmittel) bzw. (b) mit der Hand (15 min, 20 °C, 1 g/L Feinwaschmittel) gewaschen. Nach jedem Waschzyklus wurde der Restgehalt an Wirkstoffen bestimmt. Zum Vergleich wurde die Versuchsreihe wiederum mit Strumpfhosen wiederholt, die mit einer wässrigen Dispersion der gleichen Mikrokapseln vorbehandelt worden waren. Die Ergebnisse sind in Tabelle 1 zusammengefasst.

**Tabelle 1**

| **Rest-Wirkstoffgehalt als Funktion der Waschzyklen** | | | | | | | | | | | | | | | |
|---|---|---|---|---|---|---|---|---|---|---|---|---|---|---|---|
| **Waschzyklen** | **0** | **1** | **2** | **3** | **4** | **5** | **6** | **7** | **8** | **9** | **10** | **15** | **20** | **25** | **30** |
| Wirkstoffgehalt [%-rel.] bei Maschinenwäsche | | | | | | | | | | | | | | | |
| Erfindungsgemäßes Beispiel H1 | 100 | 70 | 58 | 50 | 42 | 40 | 38 | 37 | 33 | 30 | 28 | 22 | 20 | 18 | 16 |
| Vergleichsbeispiel | 100 | 60 | 39 | 21 | 5 | 0 | | | | | | | | | |

| Wirkstoffgehalt [%-rel.] bei Handwäsche | | | | | | | | | | | | | | | |
|---|---|---|---|---|---|---|---|---|---|---|---|---|---|---|---|
| Erfindungsgemäßes Beispiel | 100 | 90 | 88 | 82 | 78 | 76 | 74 | 72 | 71 | 70 | 69 | 52 | 45 | 42 | 41 |
| Vergleichsbeispiel | 100 | 81 | 66 | 51 | 32 | 12 | 3 | 0 | | | | | | | |

Man erkennt, dass die Ausrüstung mit Mischungen aus Esterquats und Mikrokapseln dazu führt, dass der Wirkstoff sowohl bei Maschinen- als auch Handwäsche weniger rasch ausgewaschen wird.

## Patentansprüche

1. Verwendung von Tensidzubereitungen, enthaltend
(a) kationische Tenside, und
(b) Mikrokapseln mit mittleren Durchmessern im Bereich von 0,0001 bis 5 mm, bestehend aus einer Hüllmembran und einer die Wirkstoffe enthaltenden Matrix, welche **dadurch** erhältlich sind, dass man
(b11) aus Gelbildnern, Chitosanen und Wirkstoffen eine Matrix zubereitet,
(b12) gegebenenfalls die Matrix in einer Ölphase dispergiert,
(b13) die dispergierte Matrix mit wässrigen Lösungen anionischer Polymere behandelt und gegebenenfalls dabei die Ölphase entfernt.
oder
(b21) aus Gelbildnern, anionischen Polymeren und Wirkstoffen eine Matrix zubereitet,
(b22) gegebenenfalls die Matrix in einer Ölphase dispergiert,
(b23) die dispergierte Matrix mit wässrigen Chitosanlösungen behandelt und gegebenenfalls dabei die Ölphase entfernt.
oder
(b31) wässrige Wirkstoffzubereitungen mit Ölkörpern in Gegenwart von Emulgatoren zu O/W-Emulsionen verarbeitet,
(b32) die so erhaltenen Emulsionen mit wässrigen Lösungen anionischer Polymere behandelt,
(b33) die so erhaltene Matrix mit wässrigen Chitosanlösungen in Kontakt bringt und
(b34) die so erhaltenen Verkapselungsprodukte von der wässrigen Phase abtrennt,
als Textilbehandlungsmittel

2. Verwendung nach Anspruch 1, **dadurch gekennzeichnet, dass** sie als Komponente (a) quaternäre Ammoniumverbindungen und/oder Esterquats enthalten.

3. Verwendung nach Anspruch 2, **dadurch gekennzeichnet, dass** sie Esterquats der Formel **(I)** enthalten, 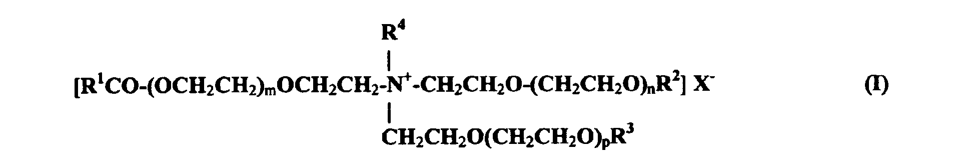 in der R¹CO für einen Acylrest mit 6 bis 22 Kohlenstoffatomen, R² und R³ unabhängig voneinander für Wasserstoff oder R¹CO, R⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht.

4. Verwendung nach Anspruch 2, **dadurch gekennzeichnet, dass** sie Esterquats der Formel (II) enthalten, 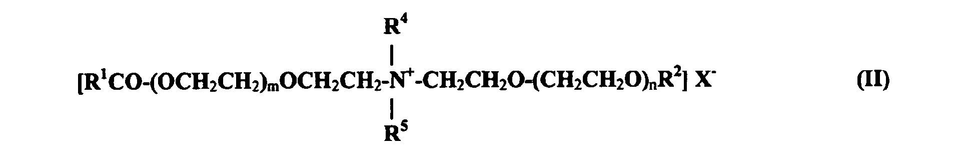 in der R¹CO für einen Acylrest mit 6 bis 22 Kohlenstoffatomen, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

5. Verwendung nach Anspruch 2, **dadurch gekennzeichnet, dass** sie Esterquats der Formel **(III)** enthalten, 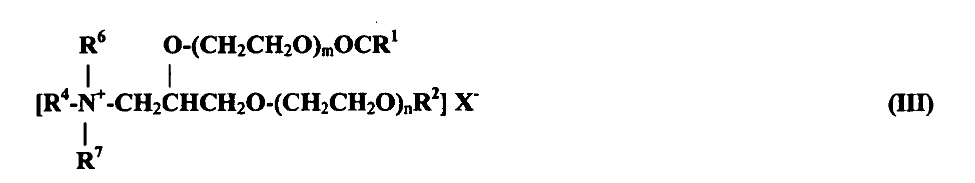 in der R¹CO für einen Acylrest mit 6 bis 22 Kohlenstoffatomen, R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

6. Verwendung nach Anspruch 2, **dadurch gekennzeichnet, dass** sie Esterquats der Formel **(IV)** enthalten, 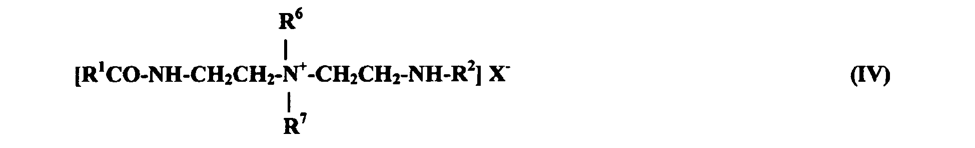 in der R¹CO für einen Acylrest mit 6 bis 22 Kohlenstoffatomen, R² für Wasserstoff oder R¹CO, R⁶ und R⁷ unabhängig voneinander für Alkylreste mit 1 bis 4 Kohlenstoffatomen und X für Halogenid, Alkylsulfat oder Alkylphosphat steht.

7. Verwendung nach Anspruch 2, **dadurch gekennzeichnet, dass** sie Esterquats der Formel (V) enthalten, 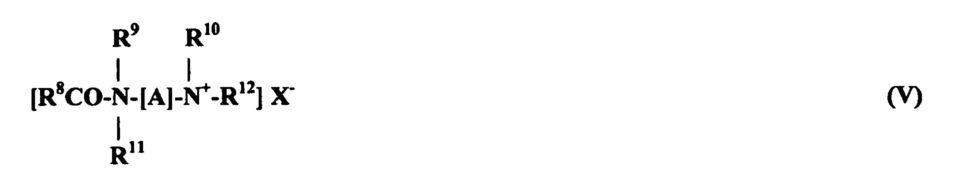 in der R⁸CO für einen gesättigten und/oder ungesättigten ethoxylierten Hydroxyacylrest mit 16 bis 22, vorzugsweise 18 Kohlenstoffatomen sowie 1 bis 50 Oxyethyleneinheiten, A für einen linearen oder verzweigten Alkylenrest mit 1 bis 6 Kohlenstoffatomen, R⁹, R¹⁰ und R¹¹ unabhängig voneinander für Wasserstoff oder eine Alkylgruppe mit 1 bis 4 Kohlenstoffatomen, R¹² für einen Alkylrest mit 1 bis 4 Kohlenstoffatomen oder einen Benzylrest und X für Halogen, Alkylsulfat oder Alkylphosphat steht.

8. Verwendung nach mindestens einem der Ansprüche 1 bis 7, **dadurch gekennzeichnet, dass** sie als Komponente (b) mikroverkapselte Wirkstoffe enthalten, die ausgewählt sind aus der Gruppe, die gebildet wird von biogenen Wirkstoffen, Antioxidantien, UV-Lichtschutzfiltern, Deodorantien, Parfümölen und Aromen.

9. Verwendung nach Anspruch 8, **dadurch gekennzeichnet, dass** sie als Komponente (b) Tocopherol, Tocopherolacetat, Tocopherolpalmitat, Carotine, Koffein, Ascorbinsäure, (Desoxy)Ribonukleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, Chitosan, Dihydroxyaceton, Menthol, Squalan, essentielle Öle (z.B. Jojobaöl), pflanzliche Proteine und deren Hydrolyseprodukte, Pflanzenextrakten, Ambroxan sowie deren Gemische enthalten.

10. Verwendung nach mindestens einem der Ansprüche 1 bis 9, **dadurch gekennzeichnet, dass** sie
(a) 10 bis 90 Gew.-% kationische Tenside und
(b) 1 bis 30 Gew.-% mikroverkapselte Wirkstoffe
mit der Maßgabe enthalten, dass sich die Mengenangaben gegebenenfalls mit Wasser, niederen aliphatischen Alkoholen oder Polyalkylenglykolen und/oder polymeren Verdickungsmitteln zu 100 Gew.-% ergänzen.

## Claims

1. Use of surfactant preparations containing
(a) cationic surfactants and
(b) microcapsules with mean diameters of 0.0001 to 5 mm which consist of a membrane and a matrix containing the active components and which may be obtained by
(b11) preparing a matrix from gel formers, chitosans and active components,
(b12) optionally dispersing the matrix in an oil phase and
(b13) treating the dispersed matrix with aqueous solutions of anionic polymers and optionally removing the oil phase in the process
or
(b21) preparing a matrix from gel formers, anionic polymers and active components,
(b22) optionally dispersing the matrix in an oil phase and
(b23) treating the dispersed matrix with aqueous chitosan solutions and optionally removing the oil phase in the process
or
(b31) processing aqueous preparations of active components with oil components in the presence of emulsifiers to form o/w emulsions,
(b32) treating the emulsions thus obtained with aqueous solutions of anionic polymers,
(b33) contacting the matrix thus obtained with aqueous chitosan solutions and
(b34) removing the encapsulated products thus obtained from the aqueous phase,
as textile treatment preparations.

2. Use claimed in claim 1, **characterized in that** they contain quaternary ammonium compounds and/or esterquats as component (a).

3. Use claimed in claim 2, **characterized in that** they contain esterquats corresponding to formula (I): 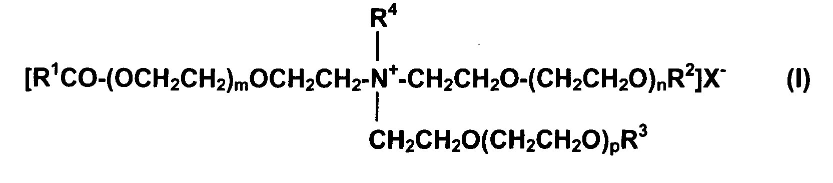 in which R¹CO is an acyl group containing 6 to 22 carbon atoms, R² and R³ independently of one another represent hydrogen or have the same meaning as R¹CO, R⁴ is an alkyl group containing 1 to 4 carbon atoms or a (CH₂CH₂O)qH group, m, n and p together stand for 0 or numbers of 1 to 12, q is a number of 1 to 12 and X is halide, alkyl sulfate or alkyl phosphate.

4. Use claimed in claim 2, **characterized in that** they contain esterquats corresponding to formula (II): 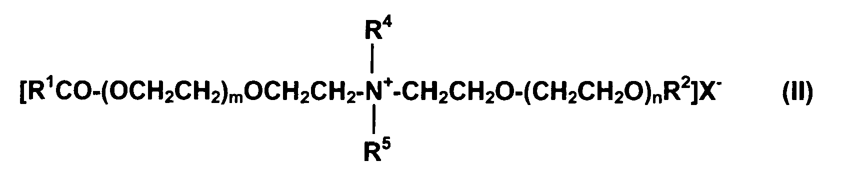 in which R¹CO is an acyl group containing 6 to 22 carbon atoms, R² is hydrogen or has the same meaning as R¹CO, R⁴ and R⁵ independently of one another are alkyl groups containing 1 to 4 carbon atoms, m and n together stand for 0 or numbers of 1 to 12 and X stands for halide, alkyl sulfate or alkyl phosphate.

5. Use claimed in claim 2, **characterized in that** they contain esterquats corresponding to formula (III): 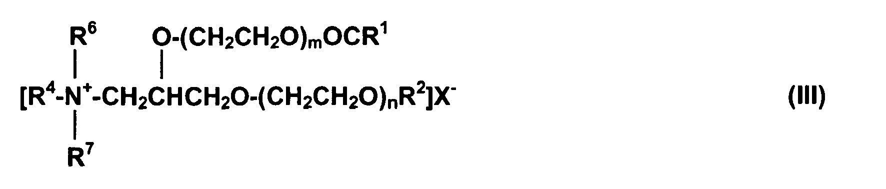 in which R¹CO is an acyl group containing 6 to 22 carbon atoms, R² is hydrogen or has the same meaning as R¹CO, R⁴, R⁶ and R⁷ independently of one another are alkyl groups containing 1 to 4 carbon atoms, m and n together stand for 0 or numbers of 1 to 12 and X stands for halide, alkyl sulfate or alkyl phosphate.

6. Use according to claim 2, **characterized in that** they contain esterquats corresponding to formula (IV): 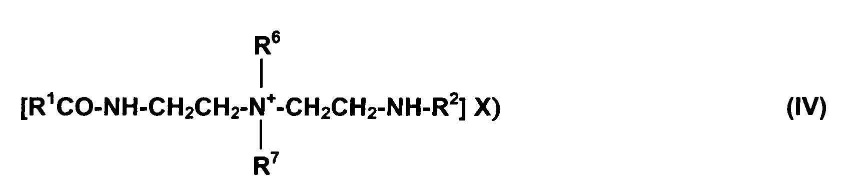 in which R¹CO is an acyl group containing 6 to 22 carbon atoms, R² is hydrogen or has the same meaning as R¹CO, R⁶ and R⁷ independently of one another are alkyl groups containing 1 to 4 carbon atoms and X is halide, alkyl sulfate or alkyl phosphate.

7. Use according to claim 2, **characterized in that** they contain esterquats corresponding to formula (V): 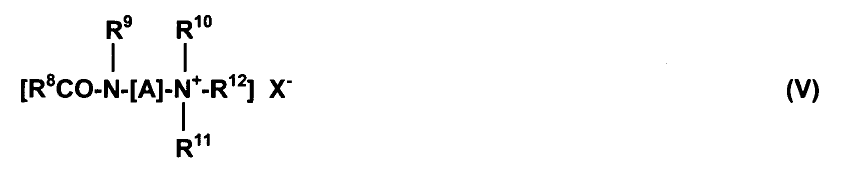 in which R⁸CO is a saturated and/or unsaturated ethoxylated hydroxyacyl group containing 16 to 22 and preferably 18 carbon atoms and 1 to 50 oxyethylene units, A is a linear or branched alkylene group containing 1 to 6 carbon atoms, R⁹, R¹⁰ and R¹¹ independently of one another represent hydrogen or a C₁₋₄ alkyl group, R¹² is a C₁₋₄ alkyl group or a benzyl group and X is halogen, alkyl sulfate or alkyl phosphate.

8. Use according to at least one of claims 1 to 7, **characterized in that** they contain as component (b) microencapsulated active components selected from the group consisting of biogenic active components, antioxidants, UV protection filters, deodorants, perfume oils and aromas.

9. Use according to claim 8, **characterized in that** they contain as component (b) tocopherol, tocopherol acetate, tocopherol palmitate, carotene, caffeine, ascorbic acid, (deoxy)ribonucleic acid and fragmentation products thereof, β-glucans, retinol, bisabolol, allantoin, phytantriol, panthenol, AHA acids, amino acids, ceramides, pseudoceramides, chitosan, dihydroxyacetone, menthol, squalane, essential oils (for example jojjoba oil), vegetable proteins and hydrolysis products thereof, plant extracts, ambroxan and mixtures thereof.

10. Use according to at least one of claims 1 to 9, **characterized in that** they contain
(a) 10 to 90% by weight cationic surfactants and
(b) 1 to 30% by weight microencapsulated active components,
with the proviso that the quantities indicated add up to 100% by weight, optionally with water, lower aliphatic alcohols or polyalkylene glycols and/or polymeric thickeners.

## Revendications

1. Utilisation de préparations tensioactives contenant :
(a) des tensioactifs cationiques et
(b) des microcapsules ayant un diamètre moyen de l'ordre de 0,0001 à 5 mm, constituées d'une membrane enveloppe et d'une matrice contenant les agents actifs, que l'on peut obtenir
(b11) en préparant une matrice à partir d'agents gélifiants, de chitosanes et d'agents actifs,
(b12) en dispersant le cas échéant la matrice dans une phase huileuse,
(b13) en traitant la matrice dispersée avec des solutions aqueuses de polymères anioniques et en éliminant, le cas échéant, la phase huileuse
ou
(b21) en préparant une matrice à partir d'agents gélifiants, de polymères anioniques et d'agents actifs,
(b22) en dispersant le cas échéant la matrice dans une phase huileuse,
(b23) en traitant la matrice dispersée avec des solutions aqueuses de chitosane et en éliminant, le cas échéant, la phase huileuse
ou
(b31) en transformant des préparations aqueuses d'agents actifs en émulsions H/E avec des corps huileux en présence d'émulsifiants,
(b32) en traitant les émulsions ainsi obtenues avec des solutions aqueuses de polymères anioniques,
(b33) en mettant la matrice ainsi obtenue en contact avec des solutions aqueuses de chitosane et
(b34) en séparant les produits d'encapsulation ainsi obtenus de la phase aqueuse,
en tant qu'agent de traitement pour textiles.

2. Utilisation selon la revendication 1,
**caractérisée en ce que**
les préparations contiennent, comme composant (a), des composés d'ammonium quaternaire et/ou des esters d'ammonium quaternaire.

3. Utilisation selon la revendication 2,
**caractérisée en ce que**
les préparations contiennent des esters d'ammonium quaternaire de formule (I) 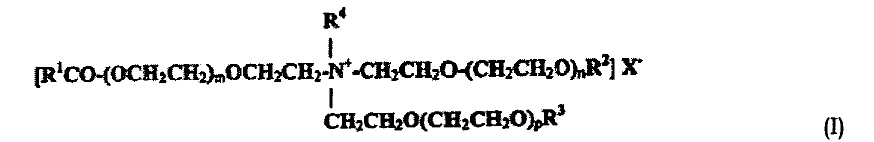 dans laquelle R¹CO représente un radical acyle comportant de 6 à 22 atomes de carbone, R² et R³ représentent indépendamment l'un de l'autre un atome d'hydrogène ou R¹CO, R⁴ représente un radical alkyle comportant de 1 à 4 atomes de carbone ou un groupe (CH₂CH₂O)_{q}H, m, n et p ont pour somme 0 ou des nombres de 1 à 12, q représente des nombres de 1 à 12, et X représente un halogénure, un sulfate d'alkyle ou un phosphate d'alkyle.

4. Utilisation selon la revendication 2,
**caractérisée en ce que**
les préparations contiennent des esters d'ammonium quaternaire de formule (II) 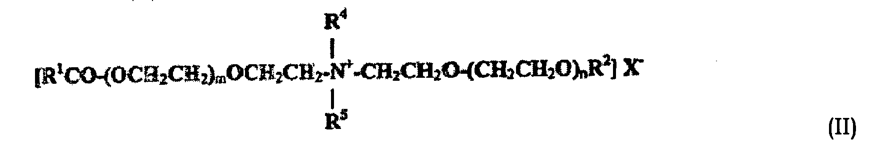 dans laquelle R¹CO représente un radical acyle comportant de 6 à 22 atomes de carbone, R² représente un atome d'hydrogène ou R¹CO, R⁴ et R⁵ représentent indépendamment l'un de l'autre des radicaux alkyle comportant de 1 à 4 atomes de carbone, m et n ont pour somme 0 ou des nombres de 1 à 12, et X représente un halogénure, un sulfate d'alkyle ou un phosphate d'alkyle.

5. Utilisation selon la revendication 2,
**caractérisée en ce que**
les préparations contiennent des esters d'ammonium quaternaire de formule (III) 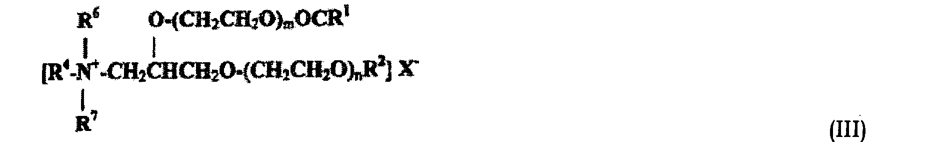 dans laquelle R¹CO représente un radical acyle comportant de 6 à 22 atomes de carbone, R² représente un atome d'hydrogène ou R¹CO, R⁴, R⁶ et R⁷ représentent indépendamment les uns des autres des radicaux alkyle comportant de 1 à 4 atomes de carbone, m et n ont pour somme 0 ou des nombres de 1 à 12, et X représente un halogénure, un sulfate d'alkyle ou un phosphate d'alkyle.

6. Utilisation selon la revendication 2,
**caractérisée en ce que**
les préparations contiennent des esters d'ammonium quaternaire de formule (IV) 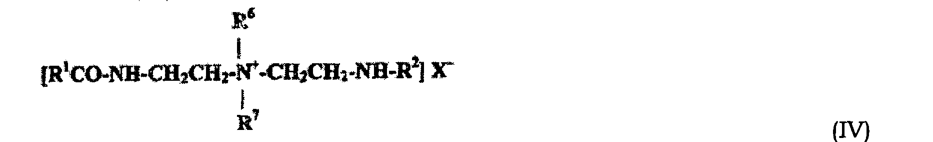 dans laquelle R¹CO représente un radical acyle comportant de 6 à 22 atomes de carbone, R² représente un atome d'hydrogène ou R¹CO, R⁶ et R⁷, représentent indépendamment l'un de l'autre, des radicaux alkyle comportant de 1 à 4 atomes de carbone, et X représente un halogénure, un sulfate d'alkyle ou un phosphate d'alkyle.

7. Utilisation selon la revendication 2,
**caractérisée en ce que**
les préparations contiennent des esters d'ammonium quaternaire de formule (V) 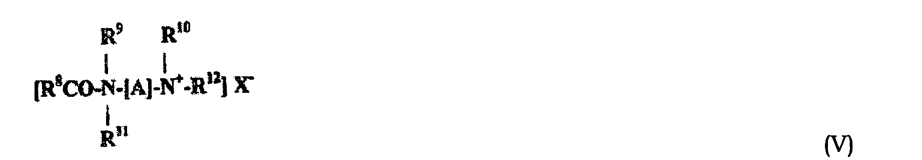 dans laquelle R⁸CO représente un radical hydroxyacyle éthoxylé, saturé et/ou insaturé, comportant de 16 à 22, de préférence 18 atomes de carbone ainsi que 1 à 50 unités d'oxyéthylène, A représente un radical alkylène linéaire ou ramifié comportant de 1 à 6 atomes de carbone, R⁹, R¹⁰ et R¹¹, représentant indépendamment les uns des autres un atome d'hydrogène ou un groupe alkyle comportant de 1 à 4 atomes de carbone, R¹² représente un radical alkyle comportant de 1 à 4 atomes de carbone ou un radical benzyle, et X représente un halogène, un sulfate d'alkyle ou un phosphate d'alkyle.

8. Utilisation selon au moins l'une des revendications 1 à 7,
**caractérisée en ce que**
les préparations contiennent, comme composant (b), des agents actifs microencapsulés choisis dans le groupe constitué d'agents actifs biogènes, d'antioxydants, de filtres de protection anti-UV, de déodorants, d'huiles parfumées et d'aromes.

9. Utilisation selon la revendication 8,
**caractérisée en ce que**
les préparations contiennent, comme composant (b), du tocophérol, de l'acétate de tocophérol, du palmitate de tocophérol, du carotène, de la caféine, de l'acide ascorbique, de l'acide (désoxy)ribonucléique et les produits de leur fragmentation, des β-glucanes, du rétinol, du bisabolol, de l'allantoïne, du phytantriol, du panthénol, des acides AHA, des acides aminés, des céramides, des pseudo-céramides, du chitosane, de la dihydroxyacétone, du menthol, du squalane, des huiles essentielles (par exemple de l'huile de jojoba), des protéines végétales et les produits de leur hydrolyse, des extraits végétaux, de l'ambroxane ainsi que leurs mélanges.

10. Utilisation selon au moins l'une des revendications 1 à 9,
**caractérisée en ce que**
les préparations contiennent :
(a) de 10 à 90 % en poids de tensioactifs cationiques et
(b) de 1 à 30 % en poids d'agents actifs microencapsulés
étant précisé que les quantités indiquées se complètent, le cas échéant, avec de l'eau, des alcools aliphatiques inférieurs ou des polyalkylène-glycols et/ou des épaississants polymères, pour obtenir 100 % en poids.
